(19) Europäisches Patentamt / European Patent Office / Office européen des brevets

(11) **EP 3 596 508 B1**

(12) **FASCICULE DE BREVET EUROPEEN**

(45) Date de publication et mention
de la délivrance du brevet:
**12.05.2021 Bulletin 2021/19**

(21) Numéro de dépôt: **18712999.4**

(22) Date de dépôt: **13.03.2018**

(51) Int Cl.:
*G01T 1/02* (2006.01)     *G01T 1/166* (2006.01)

(86) Numéro de dépôt international:
**PCT/FR2018/050583**

(87) Numéro de publication internationale:
**WO 2018/167422 (20.09.2018 Gazette 2018/38)**

(54) **MÉTHODE D'ESTIMATION DE LA DOSE DÉLIVRÉE PAR UN SYSTÈME DE RADIOLOGIE**

VERFAHREN ZUR ABSCHÄTZUNG DER ABGEGEBENEN STRAHLUNGSDOSIS IN EINEM
RADIOLOGISCHEN SYSTEM

DELIVERED-DOSE ESTIMATION METHOD BY A RADIOLOGICAL SYSTEM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.03.2017 FR 1752153**

(43) Date de publication de la demande:
**22.01.2020 Bulletin 2020/04**

(73) Titulaire: **D.R.E.A.M
Développement Et Recherches
En Applications Médicales
31300 Toulouse (FR)**

(72) Inventeur: **BOUTRY, Christine
31320 Mervilla (FR)**

(74) Mandataire: **Brevalex
56, Boulevard de l'Embouchure
B.P. 27519
31075 Toulouse Cedex 2 (FR)**

(56) Documents cités:
**EP-A2- 0 979 027     WO-A1-2015/177343**

• **WILLI A. KALENDER ET AL: "Generating and
using patient-specific whole-body models for
organ dose estimates in CT with increased
accuracy: Feasibility and validation", PHYSICA
MEDICA, vol. 30, no. 8, 1 décembre 2014
(2014-12-01), pages 925-933, XP055393945, IT
ISSN: 1120-1797, DOI:
10.1016/j.ejmp.2014.09.005**

**Description**

**DOMAINE TECHNIQUE**

La présente invention concerne de manière générale le domaine de la radiologie et plus particulièrement celui de la dosimétrie en radiologie.

**ÉTAT DE LA TECHNIQUE ANTÉRIEURE**

L'accroissement du nombre d'examens radiologiques (radiographie conventionnelle, scanographie, radiologie interventionnelle) subis par les patients est devenu un sujet majeur de santé publique. En effet, l'exposition aux rayonnements ionisants induit des dépôts d'énergie dans la matière et conduit à des effets radiobiologiques qui peuvent avoir des conséquences graves, de nature déterministe (radiodermite par exemple) ou stochastique (cancer par exemple). Il est par conséquent essentiel de pouvoir assurer une évaluation précise ainsi qu'un suivi des doses d'énergie délivrées aux patients au cours du temps.

A ce jour, il existe principalement deux solutions pour estimer une dose délivrée en radiologie. La première solution est de mesurer le produit dose surface ou PDS par une chambre d'ionisation en sortie du tube à rayons X avec détermination de la surface d'entrée de cette chambre. Toutefois, cette mesure ne prend en compte que les paramètres physiques du tube et non la morphologie du patient. Une seconde solution est de calculer la dose à l'entrée (du patient) en fonction des paramètres physiques du tube et de la géométrie d'exposition. Toutefois, ce calcul est généralement très imprécis, ne tient pas compte de la morphologie du patient et ne permet pas de prendre en compte des variations de géométrie au cours du temps (scanographie ou radiologie interventionnelle par exemple) Le document WO 2015/177343 divulgue un calcul de la dose délivrée par un système de radiothérapie à une profondeur de référence dans l'eau et en utilisant une valeur d'un niveau de gris d'un pixel central du détecteur obtenue dans des conditions de référence.

EP 0 979 027 divulgue la détermination d'une exposition aux rayonnements ou la dose absorbée par mesure du "air-Kerma area product" à l'entrée d'un patient, pendant le temps d'exposition du patient dans un système de radiologie. La valeur du niveau de air-Kerma est estimée par un réseau de neurones à partir de paramètres de la source d'exposition aux rayonnements X.

Le document, WILLI A. KALENDER ET AL: "Generating and using patient-specific whole-body models for organ dose estimates in CT with increased accuracy: Feasibility and validation",PHYSICA MEDICA, vol. 30, no. 8, 1 décembre 2014 (2014-12-01), pages 925-933, divulgue la modélisation par MC et une estimation de dose basée sur les paramètres de l'exposition, l'anatomie du patient et le transport de rayonnements dans le corps du patient. La méthode de calibration utilise des valeurs de air-Kerma mesurées avec une chambre d'ionisation.

On présentera dans un premier temps les grandeurs dosimétriques de référence.

De manière générale, l'estimation des doses en radiologie fait appel à une grandeur dosimétrique caractéristique du faisceau, dénommée Kerma (*Kinetic Energy Released per unit Mass*). Le kerma est défini par :

$$K = \frac{dE_{tr}}{dm} \qquad (1)$$

où $dE_{tr}$ est l'énergie totale transférée (sous forme d'énergie cinétique) des photons X aux particules chargées (électrons) dans un volume de matière de masse *dm*. Le kerma s'exprime en J.kg$^{-1}$.s$^{-1}$ ou, de manière équivalente en Gy. s$^{-1}$. Le kerma est généralement constitué de deux composantes : le kerma de collision ($K_{col}$) et le kerma de radiation ($K_{rad}$) mais dans la gamme d'énergie des photons X pour la radiologie le kerma de radiation peut être négligé par rapport au kerma de collision.

En radiologie conventionnelle, le kerma dans l'air peut être exprimé à partir des paramètres d'exposition du tube à rayons X. Plus précisément, le kerma dans l'air, en un point P donné, $K_{air}(P)$, est proportionnel à la charge du tube utilisée, *mAs,* (c'est-à-dire le produit de l'intensité du courant traversant le tube par le temps d'exposition) et au carré de la tension accélératrice ($kV_p$) des électrons dans le tube. Il est par ailleurs inversement proportionnel au carré de la distance ($d_{FP}$) du point en question au foyer de la source et à un facteur de filtrage (*F*) du tube , soit :

$$K_{air}(P) \propto \frac{mAs}{d_{FP}^2} . \frac{\left(kV_p\right)^2}{F} \qquad (2)$$

La dose absorbée représente l'énergie déposée localement par unité de masse *dm.* Elle est définie par :

$$D = \frac{d\varepsilon}{dm} \tag{3}$$

où $d\varepsilon$ est l'énergie déposée par rayonnement ionisant (direct ou indirect) dans un élément de matière de masse *dm.* Elle s'exprime en J.kg$^{-1}$ ou, de manière équivalente, en Gy.

Comme indiqué précédemment, le kerma *K* représente l'énergie transférée alors que la dose absorbée, *D*, représente l'énergie absorbée. En règle générale, l'énergie n'est pas absorbée à l'endroit où elle est déposée en raison du parcours des électrons secondaires dans le matériau. Toutefois, dans le domaine de la radiologie, les photons X étant de basse énergie (énergie inférieure à 150 keV), l'équilibre électronique s'établit à une faible profondeur dans le matériau et il est communément admis que $D \approx K$.

Un certain nombre de grandeurs dosimétriques, dites dérivées, sont obtenues à partir des grandeurs dosimétriques de référence. Il s'agit notamment du débit de kerma dans l'air normalisé, noté $nK_{air}$. Cette grandeur, encore appelée rendement du tube, représente le kerma par unité de charge électrique passant dans le tube et est donc définie par :

$$nK_{air} = \frac{K_{air}}{mAs} \tag{4}$$

où $nK_{air}$ s'exprime en $Gy.(mAs)^{-1}$.

On trouvera une description détaillée des grandeurs dosimétriques et de leurs relations dans le rapport de la Société Française de Physique Médicale, SFPM n°30, décembre 2014, intitulé « Dosimétrie des explorations diagnostiques en radiologie ».

Différents types de dosimètres ont été envisagés dans la littérature pour estimer la dose absorbée lors d'un examen radiologique. En particulier, la demande FR-A-2849697 propose d'utiliser un réseau de fibres optiques croisées couplées à un photomultiplicateur multi-canaux pour mesurer une dose lors d'une procédure radiologique interventionnelle. Toutefois, ce dispositif est complexe et coûteux, et ne permet pas de mesurer avec justesse la dose absorbée par le patient.

Le but de la présente invention est par conséquent de proposer une méthode de mesure qui soit simple, peu coûteuse et permette d'évaluer la dose absorbée par le patient avec une marge d'erreur sensiblement inférieure à celle de l'état de la technique.

**EXPOSÉ DE L'INVENTION**

La présente invention est définie par une méthode d'estimation de dose délivrée à un patient par un système de radiologie équipé d'une source de rayons X et d'un capteur plan, selon laquelle :

(a) on acquiert une image radiologique du patient au moyen du capteur plan;
(b) on détermine le niveau de gris, *NG,* du pixel au centre de l'image radiologique, correspondant à l'intersection de l'image avec l'axe du faisceau de rayons X ;
(c) on calcule le kerma $K_e$ à l'entrée du patient à partir dudit niveau de gris, *NG* , de la charge électrique traversant le tube de rayons X de la source, *mAs*, pendant le temps d'exposition du patient, de l'épaisseur $\delta$ du corps du patient selon l'axe du faisceau de rayons X et de la distance $d_i$ séparant la source de rayons X du patient.

Le kerma à l'entrée du patient peut alors être calculé au moyen de l'expression :

$$K_e = B \left( \frac{d_{ref}}{d_i} \right)^2 \left[ a + b \ln \left( \frac{NG}{mAs} . e^{\mu\delta} - c \right) \right] mAs$$

où *B* est un facteur de rétrodiffusion prédéterminé, $d_{ref}$ est une distance de référence à laquelle est effectuée la calibration de dosimétrie, *a,b,c* sont des coefficients d'une loi de calibration du débit de kerma dans l'air à ladite distance de référence et $\mu$ est le coefficient d'atténuation des rayons X dans l'eau.

Avantageusement, lors d'une première phase de calibration, on mesure, pour une pluralité de valeurs d'ampérage du tube et/ou de la tension accélératrice et/ou du temps d'exposition, d'une part le débit de kerma dans l'air, $nK_{air}$, à ladite distance de référence $d_{ref}$ au moyen d'un dosimètre absolu et, d'autre part, le niveau de gris du pixel au centre de l'image radiologique, normalisé par la charge électrique traversant le tube de rayons X, les coefficients $a,b,c$ étant déterminés comme ceux vérifiant au mieux la relation $nK_{air} = a + b\ln(NGn - c)$ pour ladite pluralité de valeurs.

Dans une seconde phase de calibration, on interpose ensuite des fantômes d'eau de différentes épaisseurs $x$ entre la source de rayons X et le capteur plan, et l'on détermine pour chacune de ces épaisseurs le niveau de gris du pixel central de l'image radiologique , $NG(x)$, ainsi que le niveau de gris de ce pixel en absence de fantôme, $NGn(0)$, pour une même charge électrique traversant le tube de rayons X, puis l'on calcule les rapports logarithmiques

$$\ln\frac{NGn(x)}{NGn(0)}$$ pour les différentes épaisseurs, et enfin on en déduit le coefficient d'atténuation par régression linéaire sur ces rapports logarithmiques.

Selon une variante, préalablement à l'étape (b), on élimine de l'image radiologique le bruit de fond en lui soustrayant une image radiologique acquise en absence d'irradiation, pour obtenir une image radiologique débruitée.

De sucroît, préalablement à l'étape (b) on corrige avantageusement les variations de gain pixel par pixel à partir de la dite image débruitée, de manière à obtenir une image débruitée d'intensité uniforme pour une irradiation uniforme du capteur plan.

## BRÈVE DESCRIPTION DES DESSINS

D'autres caractéristiques et avantages de l'invention apparaîtront à la lecture d'un mode de réalisation préférentiel de l'invention en référence aux figures jointes parmi lesquelles :

La Fig. 1A représente de manière de manière schématique une première configuration d'un système de radiologie lors d'une première étape de calibration de la méthode de dosimétrie selon l'invention ;
La Fig. 1B représente de manière schématique une deuxième configuration d'un système de radiologie lors d'une seconde étape de calibration de la méthode de dosimétrie selon l'invention ;
La Fig. 1C représente de manière schématique une troisième configuration d'un système de radiologie lors de la méthode de dosimétrie selon l'invention ;
La Fig. 2 représente de manière schématique un organigramme de la méthode de de dosimétrie pour un système de radiologie, selon un mode de réalisation de l'invention.

## EXPOSÉ DÉTAILLÉ DE MODES DE RÉALISATION PARTICULIERS

L'idée à la base de l'invention est d'évaluer la dose délivrée à un patient à partir du niveau de gris au centre de l'image radiologique. Par centre de l'image radiologique, on entend le point d'intersection de l'axe du faisceau de rayons X avec le plan de l'image.

L'image radiologique est obtenue au moyen d'un capteur plan capable de détecter un rayonnement X incident à faible énergie et de l'échantillonner à la fois spatialement et en intensité.

On distingue les capteurs plans à conversion directe et les capteurs plans à conversion indirecte. Les capteurs plans à conversion directe utilisent un matériau photoconducteur (par exemple le sélénium amorphe) pour convertir directement le rayonnement X incident en charges électriques qui sont collectées par une matrice de transistors TFT. Les capteurs plans à conversion indirecte utilisent l'association d'un scintillateur et d'une matrice active de photodiodes (en silicium amorphe), le scintillateur convertissant les photons X en photons optiques et la matrice active convertissant les photons optiques en charges électriques. Les charges électriques ainsi générées sont collectées par une matrice de transistors TFT comme dans le capteur à conversion directe.

Le système d'imagerie est composé d'une source de rayonnement basse énergie (keV) et d'un capteur plan, associé avec un système d'acquisition. La source et le capteur plan sont montés sur deux bras robotiques (non représentés) et peuvent être mis en vis-à-vis.

Les bras robotiques peuvent réaliser une rotation (généralement de 360°) autour d'un point fixe appelé isocentre.

Le système d'acquisition reçoit l'image radiologique brute du capteur plan, autrement dit les valeurs d'intensité des différents pixels. Il effectue tout d'abord une correction des inhomogénéités d'offset du capteur plan, pixel par pixel. Plus précisément, il soustrait à chaque pixel l'intensité détectée en absence d'irradiation dite intensité DF (*Dark Field*). En d'autres termes, on soustrait à l'image radiologique brute une image radiologique obtenue sans irradiation de manière à obtenir une image débruitée. Il corrige ensuite, les inhomogénéités de gain, pixel par pixel, dues aux variations des paramètres des amplificateurs dans la matrice de détection, de sorte que l'image débruitée soit d'intensité uniforme pour une irradiation uniforme du capteur plan.

Un tel système d'acquisition et les corrections de gain qu'il applique sont bien connus de l'état de la technique.

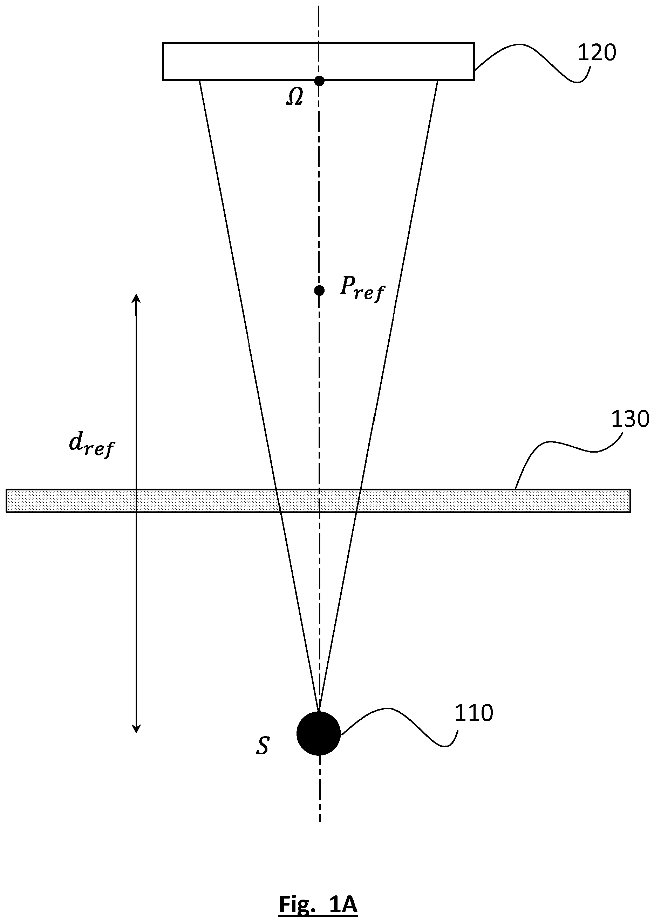

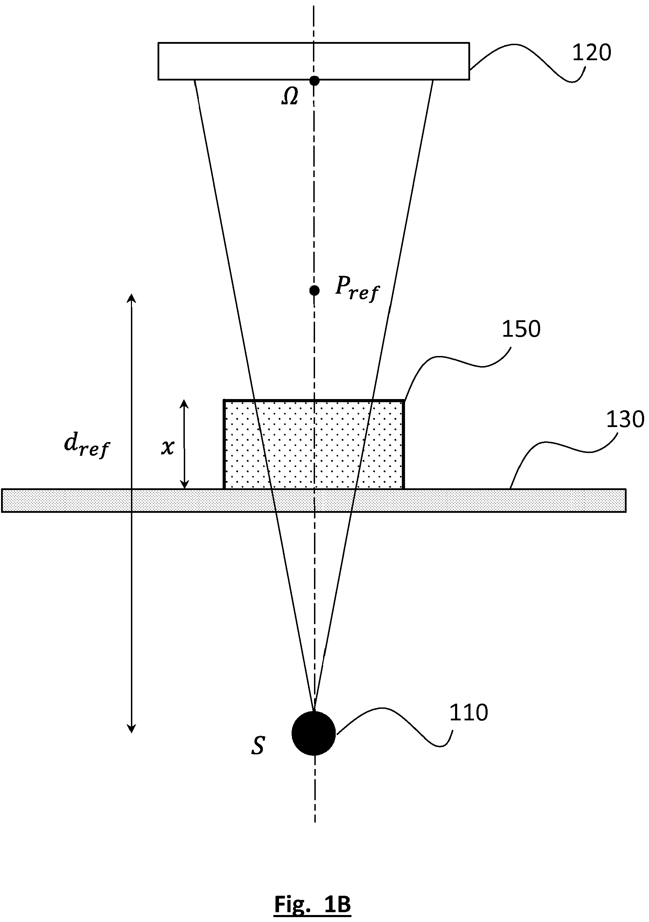

La méthode de dosimétrie selon la présente invention suppose d'effectuer préalablement des étapes de calibration qui sont décrites ci-après en relation avec les Figs. 1A et 1B.

La Fig. 1A représente de manière schématique une première configuration d'un système de radiologie lors d'une première étape de calibration de la méthode de dosimétrie.

On a désigné la source de rayons X par 110 et le capteur plan par 120. La source et le capteur plan sont montés en vis-à-vis sur un bras robotisé. On a illustré en 130 une table sur laquelle peut être allongé le patient durant l'intervention. On comprendra cependant que cette table est facultative et qu'elle peut être en outre remplacée par un autre type de support permettant de positionner le patient. On supposera dans la suite que la table présente une faible atténuation aux rayons X basse énergie (keV), de sorte que son influence pourra être négligée en première approximation.

Lors de la première étape de calibration, les mesures sont effectuées « à feu nu », c'est-à-dire sans atténuation entre la source et le capteur plan, autrement dit en absence de patient ou de fantôme de calibration.

On a pu montrer que le débit de kerma dans l'air, mesuré à une distance de référence, $d_{ref}$, de la source, pouvait s'exprimer sous la forme :

$$nK_{air} = a + b \ln \left( NGn - c \right) \qquad (5)$$

où $NGn$ est le niveau de gris du pixel au centre, $\Omega$, de l'image radiologique (fournie par le système d'acquisition), normalisé par la charge électrique passant dans le tube, soit :

$$NG_n = \frac{NG}{mAs} \qquad (6)$$

où $NG$ est le niveau de gris au centre de l'image radiologique.

Les coefficients $a,b,c$ figurant dans la loi de calibration (5) sont des constantes qui ne dépendent que des caractéristiques du système de radiologie (source, capteur plan).

Les coefficients $a,b,c$ sont calculés à partir d'une pluralité de mesures de niveaux de gris normalisés ($NGn$) pour différents débits de kerma ($nK_{air}$), en minimisant l'erreur quadratique entre les mesures et la courbe représentant la loi de calibration (*curve fitting*). Les différents débits de kerma normalisés peuvent être obtenus par exemple en faisant varier l'ampérage du tube, la tension accélératrice et/ou le temps d'exposition.

On comprendra que la loi de calibration (5) permet de relier un niveau de gris de l'image radiologique et à un débit de kerma mesuré dans l'air, à une valeur de référence.

La mesure de débit de kerma à la distance de référence peut être réalisée par exemple au moyen d'un dispositif de mesure de dose tel que le kit NOMEX™ de la société PTW, ayant bénéficié d'un étalonnage primaire.

La Fig. 1B représente de manière schématique une seconde configuration d'un système de radiologie lors d'une seconde étape de calibration de la méthode de dosimétrie.

Cette seconde configuration se distingue de la première par l'interposition d'un fantôme d'eau, 150, entre la source et le capteur plan, les autres éléments du système de radiologie restant identiques.

Si l'on note $x$ l'épaisseur du fantôme dans l'axe du faisceau, l'intensité $I(x)$ du faisceau atteignant le capteur plan au centre de l'image radiologique, après avoir traversé le fantôme d'eau, est donnée par :

$$I(x) = I_0 e^{-\mu x} \qquad (7)$$

où $\mu$ est le coefficient d'atténuation du faisceau dans l'eau et $I_0$ est l'intensité du faisceau au centre de l'image radiologique à feu nu (en absence de fantôme). On peut donc en déduire :

$$NGn(x) = NGn(0) e^{-\mu x} \qquad (8)$$

où $NGn(x)$ et $NGn(0)$ sont les niveaux de gris normalisés du pixel au centre de l'image radiologique, $\Omega$, respectivement en présence de fantôme d'eau et à feu nu.

En utilisant des fantômes d'eau de différentes épaisseurs $x$ et en calculant pour ces différentes épaisseurs les rapports logarithmiques $\ln \dfrac{NGn(x)}{NGn(0)}$, on peut en déduire par régression linéaire le coefficient d'atténuation, $\mu$. Alter-

nativement, on pourra effectuer la régression linéaire sur les rapports logarithmiques $\ln\dfrac{NG(x)}{NG(0)}$ à condition de mesurer les niveaux de gris $NG(x)$ et $NG(0)$ pour une même charge électrique traversant le tube de rayons X.

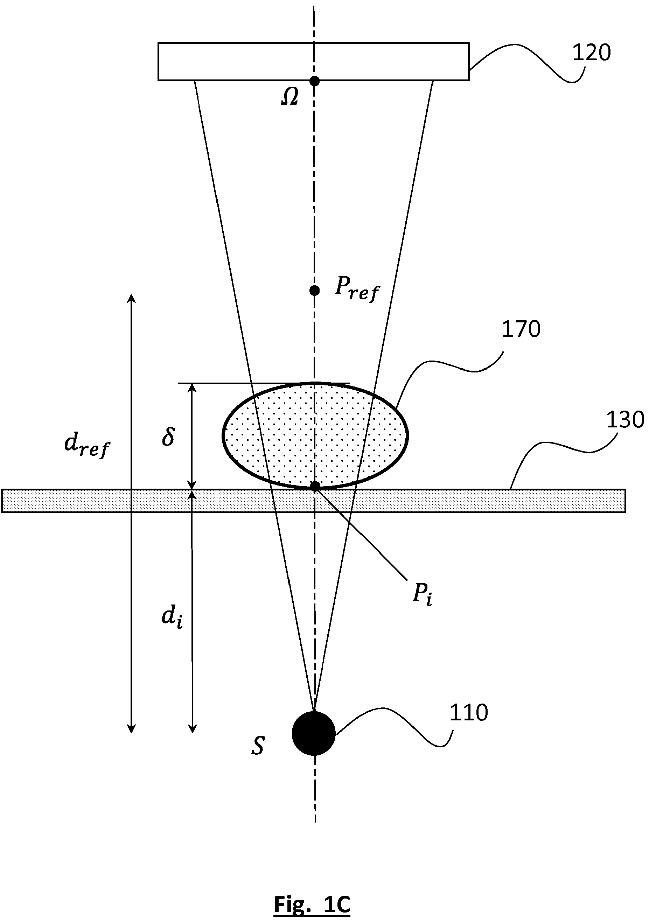

La Fig. 1C représente de manière schématique une troisième configuration du système de radiologie lors de la mesure de dose délivrée à un patient, conformément à un mode de réalisation de la méthode de dosimétrie selon l'invention.

La troisième configuration est identique à la seconde configuration à la différence près que le patient 170 est disposé entre la source de rayons X et le capteur plan, en lieu et place du fantôme d'eau.

Le corps humain étant essentiellement constitué d'eau, on peut considérer en première approximation que le coefficient d'atténuation des rayons X dans le patient est égal au coefficient d'atténuation $\mu$ obtenu dans la seconde étape de calibration.

A partir du niveau de gris du pixel au centre, $\Omega$, de l'image radiologique du patient, on peut déterminer le débit de kerma dans l'air, mesuré à la distance de référence par :

$$nK_{air} = a + b\ln\left(NGn(0) - c\right) = a + b\ln\left(NGn(e)e^{\mu\delta} - c\right) \qquad (9)$$

où $NGn(\delta)$ est le niveau de gris normalisé du pixel en question, $\delta$ étant l'épaisseur du corps du patient dans l'axe du faisceau.

Le kerma dans l'air à la distance de référence de la source est donc donné par :

$$K_{air} = \left[a + b\ln\left(NGn(\delta)e^{\mu\delta} - c\right)\right]mAs \qquad (10)$$

où $mAs$ est la charge électrique traversant le tube pendant le temps d'exposition. On peut en déduire le kerma, $K_i$, au point de contact $P_i$ entre le corps du patient et la table, sur l'axe du faisceau. Si l'on note $d_i$ la distance entre la source de rayons X et le point de contact $P_i$, on a, d'après la relation (2) :

$$K_i = K_{air}\cdot\left(\frac{d_{ref}}{d_i}\right)^2 \qquad (11)$$

Le kerma à l'entrée du patient, ou dose délivrée au patient, est la grandeur dosimétrique en radiologie conventionnelle. Elle prend en compte la dose, $K_i$, déposée par le faisceau incident à la surface du patient mais aussi la dose déposée par le rayonnement rétrodiffusé à la surface du patient. On admet généralement que le kerma à l'entrée du patient, $K_e$, est le produit du kerma dû au faisceau incident par un facteur de rétrodiffusion, $B$, soit :

$$K_e = K_i.B \qquad (12)$$

En définitive, le kerma à l'entrée du patient peut s'exprimer sous la forme suivante :

$$K_e = B\left(\frac{d_{ref}}{d_i}\right)^2\left[a + b\ln\left(\frac{NG}{mAs}.e^{\mu\delta} - c\right)\right]mAs \qquad (13)$$

On rappelle que les coefficients $a,b,c$ sont obtenus par la première étape de calibration et que le coefficient d'atténuation est obtenu par la seconde étape de calibration. La charge électrique $mAs$ passant par le tube est donnée comme le produit de l'ampérage traversant le tube et du temps d'exposition.

L'épaisseur $\delta$ pourra être estimée à partir de la corpulence du patient. Alternativement, elle pourra être estimée au moyen d'un dispositif de mesure de distance (équipé d'un capteur à ultrasons ou de lumière IR) intégré dans le capteur plan. Dans ce cas, si l'on note $d_{SD}$ la distance entre la source et le détecteur (capteur plan) et $z$ la distance entre le capteur plan et le corps du patient selon l'axe du faisceau, mesurée par le dispositif en question, on aura

simplement :

$$\delta = d_{SD} - d_i - z \qquad (14)$$

Le facteur de rétrodiffusion est généralement fixé à une valeur moyenne de référence (typiquement *B* est choisi égal à 1.35 environ), ou bien tiré de tables obtenues par des simulations Monte-Carlo pour des qualités de faisceaux données.

Le niveau de gris du pixel au centre de l'image radiologique, *NG,* est fourni par le capteur plan ou, lorsque celui-ci existe, par le système d'acquisition.

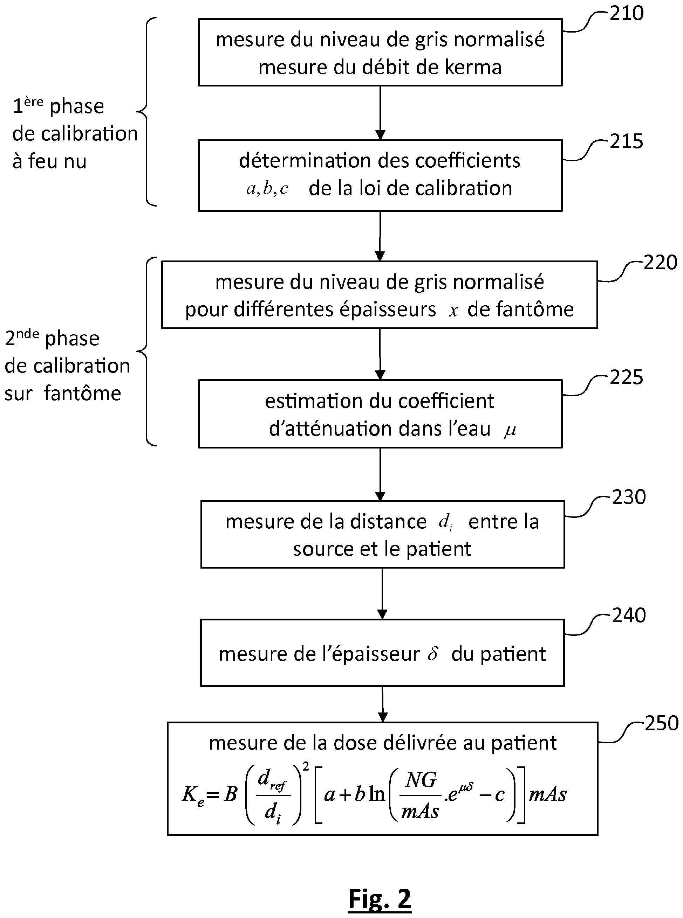

La Fig. 2 représente de manière schématique un organigramme de la méthode de de dosimétrie pour un système de radiologie, selon un mode de réalisation de l'invention.

Les étapes 210 à 225 sont des étapes de calibration proprement dites et effectuées préalablement à la mesure de dose proprement dite.

En 210, dans une configuration du système à feu nu, on effectue des mesures de niveau de gris du pixel au centre, Ω, de l'image radiologique, ce pour différentes valeurs d'ampérage du tube, de tension accélératrice et/ou différents temps d'exposition, et l'on en déduit les niveaux de gris normalisés correspondants, *nNG.*

Corrélativement, on mesure, au moyen d'un dosimètre absolu, le kerma dans l'air à une distance de référence, $d_{ref,}$ de la source de rayons X, et l'on en déduit le débit de kerma $nK_{air} = \dfrac{K_{air}}{mAs}$ , dans les mêmes conditions.

A l'étape 215, on déduit des mesures de $nK_{air}$ et *nNG,* les coefficients de la loi de calibration (5).

A l'étape 220, on mesure le niveau de gris normalisé du pixel au centre, Ω, de l'image radiologique, *NGn,* pour des fantômes d'eau de différentes épaisseurs *x* interposés entre la source de rayons X et le capteur plan, selon la seconde configuration du système de radiologie.

A l'étape 225, on déduit des mesures *NGn* précédentes, le coefficient d'atténuation dans l'eau, $\mu$, des rayons X émis par la source.

Dans la suite le patient est interposé entre la source et le capteur plan.

A l'étape 230, on mesure la distance $d_i$ de la source au patient le long de l'axe du faisceau.

A l'étape 240, on estime ou l'on mesure l'épaisseur $\delta$ du patient le long de l'axe du faisceau.

Enfin, à l'étape 250, on estime la dose délivrée au patient à partir du niveau de gris du pixel au centre de l'image radiologique, *NG,* au moyen de l'expression (13).

On a pu montrer que les doses mesurées à partir du niveau de gris *NG* étaient très proches des valeurs réelles, avec une marge d'erreur d'au plus 5%-10% environ, soit sensiblement moins que les marges d'erreur généralement constatées pour les méthodes de dosimétrie de l'état de la technique (30%).

De manière plus générale, l'homme du métier comprendra qu'après la phase de calibration, la méthode exposée plus haut permet de convertir une image radiologique, en niveaux de gris, fournie par le système d'acquisition, en image en dose. Pour ce faire, on peut remplacer dans l'expression (13), l'épaisseur $\delta$ selon l'axe du faisceau par l'épaisseur $\delta/\cos\theta$ où $\theta$ est l'angle que fait le rayon joignant la source et le pixel avec l'axe du faisceau. L'image en dose ainsi calculée donne en chaque point la dose délivrée au patient.

## Revendications

1. Méthode d'estimation de dose délivrée à un patient par un système de radiologie équipé d'une source de rayons X et d'un capteur plan, **caractérisée en ce que** :

   (a) on acquiert une image radiologique du patient au moyen du capteur plan;
   (b) on détermine le niveau de gris, *NG,* du pixel au centre de l'image radiologique, correspondant à l'intersection de l'image avec l'axe du faisceau de rayons X ;
   (c) on calcule le kerma $K_e$ à l'entrée du patient à partir dudit niveau de gris, *NG* , de la charge électrique traversant le tube de rayons X de la source, *mAs*, pendant le temps d'exposition du patient, de l'épaisseur $\delta$ du corps du patient selon l'axe du faisceau de rayons X et de la distance $d_i$ séparant la source de rayons X du patient.

2. Méthode d'estimation de dose délivrée à un patient selon la revendication 1, **caractérisée en ce que** le kerma à l'entrée du patient est calculé au moyen de l'expression :

$$K_e = B \left( \frac{d_{ref}}{d_i} \right)^2 \left[ a + b \ln \left( \frac{NG}{mAs} . e^{\mu\delta} - c \right) \right] mAs$$

où $B$ est un facteur de rétrodiffusion prédéterminé, $d_{ref}$ est une distance de référence à laquelle est effectuée la calibration de dosimétrie, $a,b,c$ sont des coefficients d'une loi de calibration du débit de kerma dans l'air à ladite distance de référence et $\mu$ est le coefficient d'atténuation des rayons X dans l'eau.

3. Méthode d'estimation de dose délivrée à un patient selon la revendication 2, **caractérisée en ce que**, lors d'une première phase de calibration, on mesure, pour une pluralité de valeurs d'ampérage du tube et/ou de la tension accélératrice et/ou du temps d'exposition, d'une part le débit de kerma dans l'air, $nK_{air}$, à ladite distance de référence $d_{ref}$ au moyen d'un dosimètre absolu et, d'autre part, le niveau de gris du pixel au centre de l'image radiologique, normalisé par la charge électrique traversant le tube de rayons X, les coefficients $a,b,c$ étant déterminés comme ceux vérifiant au mieux la relation $nK_{air} = a + b \ln(NGn - c)$ pour ladite pluralité de valeurs.

4. Méthode d'estimation de dose selon la revendication 2 ou 3, **caractérisée en ce que**, dans une seconde phase de calibration, on interpose des fantômes d'eau de différentes épaisseurs x entre la source de rayons X et le capteur plan, que l'on détermine pour chacune de ces épaisseurs le niveau de gris du pixel central de l'image radiologique , $NG(x)$, ainsi que le niveau de gris de ce pixel en absence de fantôme, $NGn(0)$, pour une même charge électrique traversant le tube de rayons X, que l'on calcule les rapports logarithmiques $\ln \frac{NGn(x)}{NGn(0)}$ les différentes épaisseurs, et que l'on en déduit le coefficient d'atténuation par régression linéaire sur ces rapports logarithmiques.

5. Méthode d'estimation de dose selon l'une des revendications précédentes, **caractérisée en ce que**, préalablement à l'étape (b), on élimine de l'image radiologique le bruit de fond en lui soustrayant une image radiologique acquise en absence d'irradiation, pour obtenir une image radiologique débruitée.

6. Méthode d'estimation de dose selon la revendication 5, **caractérisée en ce que**, préalablement à l'étape (b), on corrige les variations de gain pixel par pixel à partir de la dite image débruitée, de manière à obtenir une image débruitée d'intensité uniforme pour une irradiation uniforme du capteur plan.

**Patentansprüche**

1. Verfahren zum Abschätzen der Dosis, die einem Patienten von einem Röntgensystem, das mit einer Röntgenquelle und einem Flachbilddetektor ausgestattet ist, zugeführt wird, **dadurch gekennzeichnet, dass**

   (a) ein Röntgenbild des Patienten mit Hilfe des Flachbilddetektors aufgenommen wird;
   (b) die Graustufe $NG$ des Pixels in der Mitte des Röntgenbildes, das dem Schnittpunkt des Bildes mit der Achse des Röntgenstrahlbündels entspricht, bestimmt wird;
   (c) die Kerma $K_e$ am Eingang des Patienten aus der Graustufe $NG$, der elektrischen Ladung $mAs$ durch die Röntgenröhre der Quelle während der Bestrahlungszeit des Patienten, der Dicke $\delta$ des Körpers des Patienten entlang der Röntgenstrahlachse und dem Abstand $d_i$ zwischen der Röntgenquelle und dem Patienten berechnet wird.

2. Verfahren zur Abschätzung der an einen Patienten abgegebenen Dosis nach Anspruch 1, **dadurch gekennzeichnet, dass** die Kerma am Eingang des Patienten berechnet wird mit Hilfe des Ausdrucks:

$$K_e = B \left( \frac{d_{ref}}{d_i} \right)^2 \left[ a + b \, ln \left( \frac{NG}{mAs} \cdot e^{\mu\delta} - c \right) \right] mAs$$

worin $B$ ein vorbestimmter Rückstreufaktor ist, $d_{ref}$ ein Referenzabstand ist, bei dem die dosimetrische Kalibrierung durchgeführt wird, $a$, $b$, $c$ Koeffizienten eines Kalibrierungsgesetzes der Luftkerma-Durchflussrate bei dem Referenzabstand sind, und $\mu$ der Abschwächungskoeffizient von Röntgenstrahlen in Wasser ist.

**3.** Verfahren zur Abschätzung der an einen Patienten abgegebenen Dosis nach Anspruch 2, **dadurch gekennzeichnet, dass** während einer ersten Kalibrierungsphase für eine Vielzahl von Werten der Röhrenstromstärke und/oder der Beschleunigungsspannung und/oder der Bestrahlungszeit einerseits die Kermaleistung in Luft $nK_{Luft}$ im genannten Referenzabstand $d_{ref}$ mittels eines absoluten Dosimeters und andererseits die Graustufe des Pixels in der Mitte des Röntgenbildes, normiert durch die elektrische Ladung, die durch die Röntgenröhre hindurchgeht, gemessen werden, wobei die Koeffizienten *a, b, c* als diejenigen bestimmt werden, die am besten die Beziehung $nK_{Luft}= a +$ *b*ln (*NGn - c*) für die Vielzahl von Werten erfüllen.

**4.** Verfahren zur Dosisabschätzung nach Anspruch 2 oder 3, **dadurch gekennzeichnet, dass** in einer zweiten Kalibrierungsphase Wasserphantome unterschiedlicher Dicke *x* zwischen der Röntgenquelle und dem Flachbilddetektor angeordnet werden, dass für jede dieser Dicken die Graustufe *NG(x)* des mittleren Pixels des Röntgenbildes sowie die Graustufe *NGn(0)* dieses Pixels bei Abwesenheit des Phantoms für die gleiche elektrische Ladung, die durch die Röntgenröhre hindurchgeht, bestimmt werden, dass die logarithmischen Verhältnisse $\ln\frac{NGn(x)}{NGn(0)}$ für die verschiedenen Dicken berechnet werden, und dass davon der Abschwächungskoeffizient durch lineare Regression an diesen logarithmischen Verhältnissen abgeleitet wird.

**5.** Verfahren zur Dosisabschätzung nach einem der vorhergehenden Ansprüche, **dadurch gekennzeichnet, dass** vor dem Schritt (b) das Hintergrundrauschen aus dem radiologischen Bild eliminiert wird, indem davon ein radiologisches Bild subtrahiert wird, das ausbleibender Bestrahlung aufgenommen wurde, um ein rauschbereinigtes radiologisches Bild zu erhalten.

**6.** Verfahren zur Dosisabschätzung nach Anspruch 5, **dadurch gekennzeichnet, dass** vor dem Schritt (b) die Verstärkungsschwankung Pixel für Pixel aus dem rauschbereinigten Bild korrigiert werden, um ein rauschbereinigtes Bild mit gleichmäßiger Intensität für eine gleichmäßige Bestrahlung des Flachbilddetektors zu erhalten.

**Claims**

**1.** Method for estimating a dose administered to a patient by a radiology system provided with a source of X rays and with a flat plate sensor, **characterised in that**:

(a) an X-ray image of the patient is acquired using the flat plate sensor;
(b) the gray scale is determined, *NG,* of the pixel at the centre of the X-ray image, corresponding to the intersection of the image with the axis of the X-ray beam;
(c) the kerma $K_e$ is calculated at the input of the patient from said gray scale, *NG,* from the electrical charge passing through the X-ray tube of the source, *mAs,* during the exposure time of the patient, from the thickness $\delta$ of the body of the patient along the axis of the X-ray beam and from the distance $d_i$ that separates the source of X rays from the patient.

**2.** Method for estimating a dose administered to a patient according to claim 1, **characterised in that** the kerma at the input of the patient is calculated using the expression:

$$K_e = B\left(\frac{d_{ref}}{d_i}\right)^2\left[a+b\ln\left(\frac{NG}{mAs}.e^{\mu\delta}-c\right)\right]mAs$$

where *B* is a predetermined backscatter factor, $d_{ref}$ is a reference distance at which the dosimetry calibration is carried out, *a,b,c* are coefficients of a calibration law of the flow rate of kerma in the air at said reference distance and $\mu$ is the coefficient of attenuation of the X rays in water.

**3.** Method for estimating a dose administered to a patient according to claim 2, **characterised in that**, during a first calibration phase, is measured, for a plurality of amperage values of the tube and/or of the accelerating voltage and/or of the exposure time, on the one hand the flow rate of kerma in the air, $nK_{air}$ at said reference distance $d_{ref}$ using an absolute dosimeter and, on the other hand, the gray scale of the pixel at the centre of the X-ray image, standardised by the electrical charge passing through the X-ray tube, with the coefficients *a,b,c* being determined as those that best satisfy the relationship $nK_{air}$ = a + $b$ln($NGn$ - c) for said plurality of values.

4. Method for estimating a dose according to claim 2 or 3, **characterised in that**, in a second calibration phase, water phantoms of different thicknesses *x* are interposed between the source of X rays and the flat plate sensor, **in that** for each one of these thicknesses the gray scale of the central pixel of the X-ray image is determined, *NG(x),* as well as the gray scale of this pixel in the absence of a phantom, *NGn(0),* for the same electrical charge passing through the X-ray tube, **in that** the logarithmic ratios $\ln\dfrac{NGn(x)}{NGn(0)}$ are calculated for the different thicknesses, and **in that** the coefficient of attenuation is deduced therefrom by linear regression on these logarithmic ratios.

5. Method for estimating a dose according to one of the preceding claims, **characterised in that**, prior to the step (b), the background noise is removed from the X-ray image by subtracting therefrom an X-ray image acquired in the absence of irradiation, in order to obtain a de-noised X-ray image.

6. Method for estimating a dose according to claim 5, **characterised in that**, prior to the step (b), the variations in gain are corrected pixel-by-pixel from said de-noised image, in such a way as to obtain a de-noised image of a uniform intensity for a uniform irradiation of the flat plate sensor.

**Fig. 1A**

**Fig. 1B**

**Fig. 1C**

**1ère phase de calibration à feu nu**

mesure du niveau de gris normalisé
mesure du débit de kerma — 210

détermination des coefficients
$a, b, c$ de la loi de calibration — 215

**2nde phase de calibration sur fantôme**

mesure du niveau de gris normalisé
pour différentes épaisseurs $x$ de fantôme — 220

estimation du coefficient
d'atténuation dans l'eau $\mu$ — 225

mesure de la distance $d_i$ entre la
source et le patient — 230

mesure de l'épaisseur $\delta$ du patient — 240

mesure de la dose délivrée au patient — 250

$$K_e = B \left( \frac{d_{ref}}{d_i} \right)^2 \left[ a + b \ln \left( \frac{NG}{mAs} . e^{\mu\delta} - c \right) \right] mAs$$

**Fig. 2**

EP 3 596 508 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015177343 A
- EP 0979027 A
- FR 2849697 A

**Littérature non-brevet citée dans la description**

- **WILLI A. KALENDER et al.** Generating and using patient-specific whole-body models for organ dose estimates in CT with increased accuracy: Feasibility and validation. *PHYSICA MEDICA,* 01 Décembre 2014, vol. 30 (8), 925-933